# EUROPEAN PATENT SPECIFICATION

(11) **EP 4 121 075 B1**
(45) Date of publication and mention of the grant of the patent: **22.07.2026**
(21) Application number: 21711279.6
(22) Date of filing: 16.03.2021
(51) Int. Cl.: A61K 36/185, A61K 31/702, A61P 9/00

(54) **COMPOSITION FOR ENHANCING UROLITHIN PRODUCTION IN A HUMAN SUBJECT**
ZUSAMMENSETZUNG ZUR VERBESSERUNG DER UROLITHIN-PRODUKTION IN EINEM MENSCHLICHEM SUBJEKT
COMPOSITION POUR AMÉLIORER LA PRODUCTION D'URROLITHINE DANS UN SUJET HUMAIN

(30) Priority: 16.03.2020 EP 20163225
(43) Date of publication of application: 25.01.2023
(73) Proprietor: Migalis ApS, 2950 Vedbæk (DK)
(72) Inventor: GIVERSEN, Ina, 2000 Frederiksberg (DK); JENSEN, Leif Helth, 6354 Vitznau (CH)
(74) Representative: AWA Denmark A/S
(86) International application number: PCT/EP2021/056615
(87) International publication number: WO 2021/185802

(56) References cited:
- US-A1- 2018 185 396
- INHAE KANG ET AL: "Improvements in Metabolic Health with Consumption of Ellagic Acid and Subsequent Conversion into Urolithins: Evidence and Mechanisms", ADVANCES IN NUTRITION, vol. 7, no. 5, 1 September 2016 (2016-09-01), United States, pages 961 - 972, XP055506316, ISSN: 2161-8313, DOI: 10.3945/an.116.012575
- SUSANNE M HENNING: "Appearance of Ellagic Acid Metabolites from Pomegranate Juice in Breast Milk: A Case Report", ANNALS OF CLINICAL CASE REPORTS, vol. 4, 30 October 2019 (2019-10-30), pages 1 - 3, XP055729392, ISSN: 2474-1655

## Description

### TECHNICAL FIELD

The present invention relates to a composition comprising 1) a preparation of *Punica granatum* L. and/or another source of ellagitannins and/or ellagic acid and 2) one or more human milk oligosaccharides (HMOs). Also disclosed herein is a method of improving the production of urolithin A and/or other urolithins in the gut by administering said composition to a human subject.

### BACKGROUND OF THE INVENTION

Urolithins are gut microbiota-derived metabolites resulting from the catabolization of ellagitannins and/or ellagic acid by the gut microbiota. Rich sources of ellagitannins are pomegranate and certain fruits and nuts, such as raspberry, strawberry, blackberry and walnut.

Ellagitannins are hydrolyzed in the gut to release ellagic acid, which is further processed by the gut microbiota into urolithins through the loss of one of its two lactones and by successive removal of hydroxyl groups.

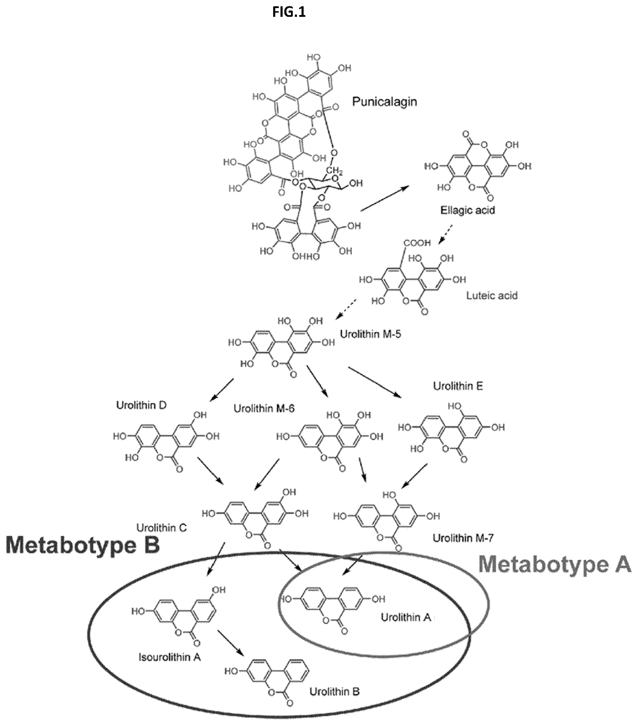

In humans, urolithin A, urolithin B and isourolithin A are the measurable final metabolites of the catabolic conversion of ellagitannins and/or ellagic acid (see Figure 1). Urolithin A is the main metabolite observed in human plasma and urine, where it mainly occurs as conjugate forms with glucuronic acid and/or sulfate.

Urolithins A, B, C and D have all shown the ability to extend the lifespan of the nematode *Chaenorhabditis elegans* (between 19.0% and 45.4%). Urolithin A activated mitophagy throughout the duration of the treatment. Mitophagy is the process by which damaged or superfluous mitochondria are removed by autophagy. The results further suggested that urolithin A treatment activated mitochondrial biogenesis in older worms. Pharyngeal pumping rate and mobility was improved by urolithin A treatment during aging. Similar effects were observed with urolithin B, whereas urolithin C and urolithin D showed mild to no benefit (Ryu et al. 2016). Treatment with urolithin A has shown improved endurance capacity (42% greater running endurance) and increased muscle function (57% greater level of spontaneous exercise) in old mice. Lean muscle mass did not increase, suggesting that urolithin A improves muscle cell quality rather than quantity. At the molecular level, treatment with urolithin A led to a general tendency for a higher expression of autophagy and mitophagy transcripts (Ryu et al. 2016).

Furthermore, urolithin A has shown promising effects on mitochondrial health in a clinical trial. Urolithin A stimulated mitochondrial biogenesis in the skeletal muscle of humans following 28 days of oral administration at doses of 500 mg/d and 1000 mg/d (Andreux et al. 2019).

Mitochondria are the central energy producers of the cell and play a vital role in a wide range of cellular processes, including regulation of cell homeostasis. An intricate regulatory network, balancing the generation of new mitochondria (mitochondrial biogenesis) and removal of damaged mitochondria (mitophagy), forms the basis of healthy aging and longevity.

Mitophagy efficiency decline with age, leading to progressive accumulation of damaged and/or superfluous mitochondria, deterioration of cellular function, and ultimately cell death. Most aging-related diseases, particularly neurodegenerative diseases, have a mitochondrial involvement. Mitochondrial dysfunction has been associated with such diverse diseases as diabetes, cancer, cardiovascular disease, Alzheimer's Disease, Parkinson's Disease, multiple sclerosis, amyotrophic lateral sclerosis, age-related macular degeneration, chronic inflammation and autoimmune disorders (Haas 2019). The significance of impaired or defective mitophagy is well-known for the development of Alzheimer's Disease (Fang et al. 2019) and Parkinson's Disease (Srivastava 2017).

Poor mitochondrial function in the skeletal muscle has been closely linked to slow walking speed and poor muscle strength in elderly individuals. Decline in muscle function in the elderly is often part of a larger syndrome, termed frailty, which is an important risk factor for disability, hospitalization and mortality. A decrease in mitophagy-related gene expression was reported to correlate with slower walking speed in frail elderly (Drummond et al. 2014).

Emerging evidence suggests that enhanced mitophagy promotes healthy aging as well as delays the onset and progression of various age-related pathologies. Thus, mitophagy modulation may serve as a potential therapeutic approach to alleviate age-associated weaknesses.

There is thus substantial evidence suggesting that urolithins have beneficial effects in relation to delaying certain aging processes linked to mitochondrial function, and therefore urolithins might be a valuable food supplement for the aging population. However, isolated urolithins may in general not be administrated directly to humans due to pending regulatory approval procedures. Urolithin A has been GRAS approved in the US, but not authorized as novel food in EU yet (Djedjibegovic et al. 2020).

An alternative to the consumption of isolated urolithins would be to use natural sources of ellagitannins such as pomegranate and certain fruits and nuts, such as raspberry, strawberry, blackberry and walnut, and rely on the *in vivo* catabolization of ellagitannins and/or ellagic acid by the gut microbiota to urolithins.

However, ingesting natural sources of ellagitannins such as pomegranate is not a generally applicable alternative, as not all individuals have the appropriate gut microbiota to produce the desired urolithin metabolites. As reported in a number of clinical trials, 5-30% of a given population are not able to convert ellagitannins or ellagic acid to urolithins, or only produce traces hereof (Tomas-Barberan et al. 2014; Li et al. 2015). Among urolithin producers, two main metabotypes have been distinguished: individuals who produce only urolithin A and individuals who produce isourolithin A and/or urolithin B in addition to urolithin A (Tomas-Barberan et al. 2014). Thus, three different urolithin metabotypes are generally referred to in the scientific literature: exclusive urolithin A-producers (UM-A), mixed urolithin producers (UM-B) and non-producers (UM-0).

It remains yet to be established which gut microorganisms are responsible for the complete catabolic conversion of ellagitannins to urolithins. A number of clinical trials and *in vitro* studies have contributed to shedding light on this question, so that at least some bacteria groups, genera and species have been recognized as playing a role in the transformation. The genus *Gordonibacter,* belonging to the family Eggerthellaceae and the phylum Actinobacteria, has been linked to urolithin production in several clinical studies conducted in Spain (González-Sarrias et al. 2017; Cortés-Martín et al. 2019; Romo-Vaquero et al. 2015).

The bacteria species *Akkermansia muciniphila* (Akkermaniaceae, Verrucomicrobia) is capable of producing ellagic acid from pomegranate ellagitannins *in vitro.* This species was found at 33-fold and 47-fold higher concentrations in stool samples of urolithin A-producers compared to non-producers at baseline and after 4 weeks of pomegranate extract administration, respectively (Li et al. 2015).

Lactic acid bacteria and *Bifidobacteria* have been reported to decrease in UM-B individuals (but not UM-A individuals) upon intake of pomegranate extract (González-Sarrias et al. 2017). On the other hand, another clinical study reported that *Bifidobacterium* increased exclusively in UM-B subjects upon walnut intake (García-Mantrana et al. 2019). Further, a higher abundance of *Bifidobacterium* (Bifidobacteriaceae, Actinobacteria) was found in UM-A postpartum women than in UM-B individuals (Cortés-Martín et al. 2019).

Similar cases of conflicting reports on gut microbiota composition under certain conditions and in certain metabotypes seem to be more the rule than the exception. In short, the breakdown of ellagitannins to urolithins has only been partly elucidated and it is likely that more routes exist, when it comes to the involved microorganisms.

At present, there is an unmet need for a means of activating urolithin production among the group of non-producers, since these individuals do not benefit from the health improving effect of urolithins.

### SUMMARY OF THE INVENTION

It is an objective of the present invention to enable urolithin non-producers, particularly men and women above 50 years of age, to benefit from the physiological advantages of urolithin in terms of healthy aging due to improved mitochondrial function. Health benefits of improved mitochondrial function include retention of muscle strength, preservation of a healthy cardiac and immune system and avoidance of neurodegenerative disorders.

It has now been found by the inventors that administering an oral composition comprising a preparation of the fruit of *Punica granatum* in combination with an effective dose of one or more human milk oligosaccharides (HMOs), leads to an activation of urolithin production in individuals who are considered "non-producers". As defined herein, non-producers do not produce any of the so-called final urolithin metabolites, i.e. urolithin A, urolithin B and isourolithin A, or only minimal amounts thereof, upon ingesting food sources containing ellagitannins and/or ellagic acid. A true non-producer cannot unequivocally be determined due to the inherit limit of detection (LoD) of analytical equipment used for the measurement of urolithins (or any other compound) in body fluids or tissue. Herein we define non-producers as individuals with a maximum amount of final urolithins in the urine of <5 µg/mg creatinine. This definition is in accordance with the findings by Li et al. (2015), *supra,* since urolithin A non-producers are technically identical with final urolithin non-producers. Li et al. (2015) reported that the group of individuals with "no baseline UA production" comprises individuals with a baseline urolithin A production up to 2.9 µg/mg creatinine in urine (Li et al., 2015). Thus, a maximum limit of 5 µg final urolithins/mg creatinine in urine seems to be a reasonable dividing line between non-producers and producers of final urolithins.

The increase in the production of urolithins following administration of an oral composition comprising a preparation of the fruit of *Punica granatum* in combination with an effective dose of one or more human milk oligosaccharides (HMOs) referred to above may not be achieved by a corresponding amount of pomegranate preparation alone or another source of ellagitannins or ellagic acid.

In a first aspect, the present invention thus provides a composition comprising a preparation of the fruit of *Punica granatum* together with an effective dose of one or more human milk oligosaccharides (HMOs). The combined effect of these substances is an increased production of final urolithins by the gut microbiota. The invention also relates to compositions for use in identification and treatment of non-producers in order to make these individuals capable of benefitting from the positive health effects of improved mitochondrial function, including increased mitophagy. This is especially true for middle-aged and elderly individuals (+50). The identification is based on analyzing a urine sample obtained from an individual after said individual has ingested a standardized dose of ellagitannins for three consecutive days.

**In a second aspect,** the present invention thus provides a composition according to the first aspect for use in the treatment of a human subject identified as being a non-producer, said identification comprising the following steps:
a) Orally administering to the human subject a standardized dose of ellagitannins for three consecutive days and,
b) Obtaining a urine sample from the human subject 72-88 hours after the human subject has ingested the first standardized dose of ellagitannins,
c) Analyzing the urine sample for the presence and levels of urolithin A, urolithin B and isourolithin A,
d) Comparing the level of urolithin A, urolithin B and isourolithin A with metabotype reference levels for UA-producers and mixed producers, respectively;
e) Determining whether the subject is a UA-producer, mixed producer or non-producer, based on the comparison of the subject levels with the reference levels,
wherein the presence of urolithin A, urolithin B and isourolithin A in a total amount less than 5µg/mg creatinine in urine classifies the subject as a non-producer.

In a third aspect, the invention also relates to compositions as described herein for use in the treatment of a human subject identified as being a non-producer, said method comprising
a) Orally administering to the human subject a standardized dose of ellagitannins for three consecutive days,
b) Obtaining a urine sample from the human subject 72-88 hours after the human subject has ingested the first standardized dose of ellagitannins,
c) Analyzing the urine sample for the presence and levels of urolithin A, urolithin B and isourolithin A,
d) Comparing the level of urolithin A, urolithin B and isourolithin A with metabotype reference levels for UA-producers and mixed producers, respectively;
e) Determining whether the subject is a UA-producer, mixed producer or non-producer, based on the comparison of the subject levels with the reference levels,
f) administering a therapeutically effective dose of a composition according to the first aspect comprising a preparation of the fruit of *Punica granatum* together with one or more human milk oligosaccharides (HMOs), to any human subject identified as belonging to non-producers; and
g) optionally monitoring said identified subject for an increase in the level of final urolithins over a period of at least 30 days, such as 30 days, 60 days or preferably 90 days.

### BRIEF DESCRIPTION OF THE FIGURES

**Figure 1****:** Gut microbiota catabolism of pomegranate ellagitannins (punicalagin) to urolithins, and differences between UA-producers (Metabotype A) and mixed producers (Metabotype B). The transient intermediate metabolite luteic acid has not yet been detected. Final urolithins are urolithin A, urolithin B and isourolithin A. All other urolithins are defined as intermediary urolithins (Garcia-Villalba et al. 2017).
**Figure 2****:** Production of urolithin A during two timeframes of the incubation (0-24h and 0-48h) upon fermentation of three different substrates, i.e. 3 g/L HMO, 1 g/L pomegranate extract (PG) and 3g/L HMO + 1g/L PG. A negative control (blank) was also included.
**Figure 3****:** Production of isourolithin A during two timeframes of the incubation (0-24h and 0-48h) upon fermentation of three different substrates, i.e. 3 g/L HMO, 1 g/L pomegranate extract (PG) and 3g/L HMO + 1g/L PG. A negative control (blank) was also included.
**Figure 4****:** Production of urolithin C during two timeframes of the incubation (0-24h and 0-48h) upon fermentation of three different substrates, i.e. 3 g/L HMO, 1 g/L pomegranate extract (PG) and 3g/L HMO + 1g/L PG. A negative control (blank) was also included.
**Figure 5****:** Average production of urolithin A across 9 donors during two timeframes of the incubation (0-24h and 0-48h) upon fermentation of three different substrates, i.e. 3 g/L HMO, 1 g/L pomegranate extract (PG) and 3g/L HMO + 1g/L PG. A negative control (blank) was also included.

### DEFINITIONS

**Autophagy:** Autophagy is the regulated digestion within a cell of cytoplasmic elements which it no longer requires.

**Bifidogenic:** A substance or compound that promotes the growth of bifidobacteria in the intestinal tract is said to be bifidogenic.

**Biogenesis:** The production of new living organisms or organelles.

**Catabolization:** The process in which molecules are broken down into smaller units through a set of metabolic pathways.

**Conjugate:** A compound formed by the joining of two or more chemical compounds.

**Decoy receptor:** A receptor that is able to recognize and bind specific growth factors or cytokines efficiently but is not structurally able to signal or activate the intended receptor complex.

**Ellagitannin:** The ellagitannins are a diverse class of hydrolysable tannins, a type of polyphenol formed primarily from the oxidative linkage of galloyl groups in 1,2,3,4,6-pentagalloyl glucose. Ellagitannins yield ellagic acid on hydrolysis. Examples of ellagitannins found in pomegranate fruit are: punicalagins A and B, punicalins, punicalin isomers.

**Final urolithins:** Final urolithins comprise urolithin A, urolithin B and isourolithin A. These urolithins are the measurable final metabolites of the catabolic conversion of ellagitannins and/or ellagic acid in the human gut.

**Fucosylated molecule:** A molecule to which one or more fucose sugar units have been added.

**Gut microbiota:** The gut microbiota is the microbial communities inhabiting the gastrointestinal tract.

**Homeostasis:** The state of steady internal, physical and chemical conditions maintained by living systems.

**Human milk oligosaccharide (HMO):** Human milk oligosaccharide, abbreviated as HMO, are complex carbohydrates found in human breast milk. The HMOs have a core structure comprising a lactose unit at the reducing end that can be elongated by one or more β-N-acetyl-lactosaminyl and/or one or more β-lacto-N-biosyl units, and which core structure can be substituted by an α-L-fucopyranosyl and/or an α-N-acetyl-neuraminyl (sialyl) moiety. In this regard, the non-acidic (or neutral) HMOs are devoid of a sialyl residue, and the acidic HMOs have at least one sialyl residue in their structure. The non-acidic (or neutral) HMOs can be fucosylated or nonfucosylated.

**Husk:** The husk is a usually dry or membranous outer covering of various seeds and fruits. The pomegranate husk is comprised of two parts: the pericarp, which provides an outer cuticle layer and fibrous mat, and the mesocarp, which is the spongy tissue and inner fruit wall where the fleshy arils are attached.

**Hydrolysis:** The chemical process in which a molecule is cleaved into two parts by the addition of a molecule of water.

**Intermediary urolithins:** Intermediary urolithins comprise urolithin M-5, urolithin M-6, urolithin M-7, urolithin C, urolithin D and urolithin E. These urolithins are the measurable intermediary metabolites of the catabolic conversion of ellagitannins and/or ellagic acid to the final metabolites urolithin A, urolithin B and isourolithin A in the human gut.

**Metabolite:** A metabolite is any substance produced during metabolism, i.e. digestion or other physiological chemical processes.

**Metabotype:** A metabolic phenotype, i.e. the kind of metabolism characterizing an individual.

**Microbiome:** The microbiome comprises all of the genetic material within a microbiota, which is the entire collection of microorganisms in a specific niche, such as the human gut.

**Mitochondria:** Mitochondria are membrane-bound cell organelles that function as power generators of the eukaryotic cell, converting oxygen and nutrients into adenosine triphosphate (ATP). Mitochondria also store calcium for cell signalling activities, generate heat and mediate cell growth and death. Mitochondria are unlike other cellular organelles in that they have two distinct membranes and a unique genome. Furthermore, they reproduce by binary fission.

**Mitophagy:** The selective degradation of dysfunctional or superfluous mitochondria by autophagy.

**Mixed producer:** An individual who produces urolithin B and/or isourolithin A in addition to urolithin A as final urolithins.

**Non-producer:** An individual who does not produce any of the final metabolites urolithin A, urolithin B or isourolithin A, or only does so in low amounts (< 5 µg/mg creatinine in urine, as defined herein).

**Pericarp:** The outer layer of a ripe fruit.

**Pomace:** The solid remains of fruits after pressing for juice or oil.

**Punicoside:** The term punicosides refers to the punicalagins and punicalins in pomegranate fruit, including punicalagin A and B, punicalin A and B and punicalin isomers.

**Prebiotics:** Dietary ingredients, usually oligosaccharides, that provide a health benefit to the host mediated by the modulation of the human gut microbiota.

**Skeletal muscle:** Skeletal muscles are the most common of the three types of muscles in the body. Most skeletal muscles are attached to bones by tendons, and they produce all the movements of body parts in relation to each other. Skeletal muscles are under voluntary control of the somatic nervous system.

**UA-producer:** an individual who only produces urolithin A as a final urolithin.

### DETAILED DESCRIPTION OF THE INVENTION

At least three types of urolithin producers or metabotypes have been documented in a number of clinical trials conducted by several research groups. Firstly, there are individuals who are described as not producing the final urolithin metabolites or even any kind of urolithins, or only traces thereof. These individuals are often referred to as "nonproducers". According to different clinical trials, the group of non-producers accounts for 5-30% of a population (Tomas-Barberan et al. 2014; Li et al. 2015). Reports of absence of excreted urolithin by individuals in this group should however be understood as an analytical issue rather than a *de facto* proof that these individuals do not produce urolithins at all (Cerdá et al. 2004; Nuñez-Sánchez et al. 2014; Truchado et al. 2012). This was pragmatically addressed in an intervention study (Li et al. 2015) in which participants with a baseline urolithin A content in urine of up to 2.9 µg/mg creatinine were classified as nonproducers.

Therefore, as mentioned above, "non-producers" are defined herein as individuals - who upon ingesting food sources containing ellagitannins and/or ellagic acid - produce the final urolithin A, urolithin B and isourolithin A in low and presumably physiologically insignificant amounts, as there seems to be no scientific rationale for claiming a complete absence of these metabolites in this group of individuals. Based on the observations by Li et al. (2015), we define non-producers as individuals with a maximum amount of final urolithins in the urine of <5 µg/mg creatinine.

Secondly, there is a predominating group of individuals who only produce urolithin A as the final metabolite. This group of exclusive urolithin A-producers, UA-producers, accounts for 25-80% of a population. Finally, a more heterogeneous group exists consisting of individuals who produce urolithin A in addition to urolithin B and/or isourolithin A. This group of mixed producers makes up 10-50% of a given population. Other metabotypes may exist, since the majority of the metabotype studies have been conducted in a Spanish population which may not be representative for all cultures and ethnic groups.

The straightforward interpretation of the occurrence of these groups is that non-producers lack one or more bacteria species or bacteria groups necessary for the efficient production of final urolithins or urolithins altogether or alternatively that these bacteria are only present at insufficiently low levels. Genetic polymorphisms or differences in the composition or functionality of the gut microbiota will also contribute to the different response of individuals to intake of ellagitannin-rich foods. In this regard, a highly variable metabolism between individuals has been described for some phenolic compounds besides ellagitannins, such as lignans and procyanidins (Ávila-Gálvez et al. 2020).

In other words, non-producers are presumably a rather heterogeneous group with various underlying causes for their insignificant production of final urolithins or urolithins altogether. The UA-producers seems to be a more homogenous group with a gut microbiota comprising all the necessary bacteria species for urolithin A production, however with variations between different populations. Finally, the group of mixed producers probably comprises at least three sub-metabotypes: production of urolithin A + isourolithin A, production of urolithin A + urolithin B and production of urolithin A + isourolithin A + urolithin B. To our knowledge, no attempt to characterize these different sub-metabotypes has been done until now.

Obviously, the non-producers are not able to benefit from the positive health effects of urolithins or only to a very limited extent. It thus seems evident that activation of an urolithin production in these individuals would be an advantage to their general health through an improved mitochondrial function. We thus find it reasonable to expect that non-producers would benefit from changing their metabotype towards a urolithin producing metabotype.

Only a few studies deal with conversions between metabotypes. The metabotype of healthy, lactating women was determined four times during the first year after having given birth (Cortés-Martín et al. 2019). It turned out that 20% of the women from the mixed producer group changed metabotype to become UA-producers for unknown reasons. Another clinical study reports the conversion of non-producers to urolithin producers among healthy overweight-obese individuals. Conversions in three out of six non-producers were observed upon intake of pomegranate extract. The circumstances of these conversions seem somewhat unclear, since the group of converted non-producers differed at baseline from obligate non-producers in their significantly higher level of *Gordonibacter.* Furthermore, the study did not report the actual urolithin production for the converted non-producers (González-Sarrías et al. 2017).

The inventors have now found that by administering a composition which comprises a preparation of the fruit of *Punica granatum,* such as a pomace extract, and one or more human milk oligosaccharides (HMOs), individuals identified as non-producers may change their metabotype to UA-producer or mixed producer, more consistently and with a significantly higher resulting urolithin production than when a corresponding amount of pomegranate extract is administrated alone.

**In a first aspect**, the present invention thus provides a composition comprising
a. a preparation of the fruit of *Punica granatum,* and
b. one or more human milk oligosaccharides (HMOs).

In an embodiment, the HMO is selected from neutral HMOs or acidic HMOs. The neutral HMO can be one or more fucosylated HMOs or one or more non-fucosylated HMOs.

Following the administration of the composition according to the first aspect, an amount equal to or higher than 5 µg/mg creatinine of one or more of the final urolithin metabolites in the urine renders a previous non-producer a UA-producer or a mixed producer.

The preparation of the fruit of *Punica granatum* is obtainable by methods known in the art. In some embodiments, the preparation of the fruit of *Punica granatum* is obtainable from the pericarp of *Punica granatum.* The preparation may be an extract of the fruit of *Punica granatum* obtained from the pomace of *Punica granatum,* i.e. by extracting the remains of squeezed fruits. The extract is preferably a dry extract, such as a powder or a granulate. Other methods of preparing an extract of the fruit of *Punica granatum* well known in the art, are also within the scope of the invention.

Specific embodiments relate to extracts of the fruit of *Punica granatum* comprising at least 40% polyphenols. The extract of the fruit of *Punica granatum* according to the present invention preferably further comprises at least 30% punicosides and/or at least 20% punicalagins.

The composition according to the first aspect of the present invention may be such that the ratio (w/w) between the preparation of the fruit of *Punica granatum* and the one or more human milk oligosaccharides (HMOs) is comprised in the range of 5:1 - 1:10.

In one embodiment, the composition according to the first aspect comprises a preparation, such as an extract, of the fruit of *Punica granatum,* one or more HMOs selected from neutral HMOs or acidic HMOs. The neutral HMO can be one or more fucosylated HMOs or one or more non-fucosylated HMOs.

**In a second aspect**, the present invention provides a composition according to the first aspect for use in the treatment of a human subject identified as being a non-producer, said identification comprising the following steps:
a) Orally administering to the human subject a standardized dose of ellagitannins for three consecutive days,
b) Obtaining a urine sample from the human subject 72-88 hours after the human subject has ingested the first standardized dose of ellagitannins,
c) Analyzing the urine sample for the presence and levels of urolithin A, urolithin B and isourolithin A,
d) Comparing the level of urolithin A, urolithin B and isourolithin A with metabotype reference levels for UA-producers and mixed producers, respectively;
e) Determining whether the subject is a UA-producer, mixed producer or non-producer, based on the comparison of the subject levels with the reference levels,
wherein the presence of urolithin A, urolithin B and isourolithin A in a total amount less than 5µg/mg creatinine in urine classifies the subject as a non-producer.

**In a third aspect,** the invention also relates to compositions as described herein for use in the treatment of a human subject identified as being a non-producer, said method comprising:
a) Orally administering to the human subject a standardized dose of ellagitannins for three consecutive days,
b) Obtaining a urine sample from the human subject 72-88 hours after the human subject has ingested the first standardized dose of ellagitannins,
c) Analyzing the urine sample for the presence and levels of urolithin A, urolithin B and isourolithin A,
d) Comparing the level of urolithin A, urolithin B and isourolithin A with metabotype reference levels for UA-producers and mixed producers;
e) Determining whether the subject is a UA-producer, mixed producer or non-producer, based on the comparison of the subject levels with the reference levels,
f) administering a therapeutically effective dose of a composition according to the first aspect comprising a preparation of the fruit of *Punica granatum* together with one or more human milk oligosaccharides (HMOs), to any human subject identified as being a non-producer; and
g) optionally monitoring said identified subject for the production of one or more final urolithins over a period of at least 30 days, such as 30 days, 60 days or preferably 90 days.

In a preferred embodiment of the second and third aspect, the treatment of individuals identified as non-producers involves administering a daily dose of the composition according to the first aspect containing between 500-10000 mg HMOs/day and 500 - 5000 mg of a preparation of the fruit of *Punica granatum*/day. Human milk oligosaccharides (HMOs) are a group of complex sugars, or oligosaccharides, that are present at high concentrations in human milk. HMOs serve as metabolic substrates for select gut microbes, thus contributing to the development of the infant gut microbiota. In other words, HMOs function as prebiotic substances.

HMOs have been found to shape the infant's gut microbiota by selectively stimulating the growth of specific bacteria, especially bifidobacteria. These bifidogenic effects of HMOs are structure-specific and may vary depending on the HMO composition in the milk of different individuals.

The structural diversity represented by HMOs can be broadly divided into fucosylated (neutral HMOs), sialyated (acidic HMOs) and non-fucosylated neutral structures. Approximately 200 different HMOs have been identified so far. The building blocks of human milk oligosaccharides are the five monosaccharides D-glucose, D-galactose, N-acetylglucosamine, L-Fucose, and sialic acid. Lactose forms the reducing end of milk oligosaccharides. The amount and composition of HMOs produced in human milk are highly variable between women.

Apart from having prebiotic effects, HMOs also act as soluble decoy receptors that block the attachment of viral, bacterial or protozoan pathogens to epithelial cell surface sugars. This function as an anti-adhesive may help prevent infectious diseases in the gut and also in the respiratory and urinary tracts.

Supplementation of 2'-fucosyllactose (2'FL) and lacto-N-neotetraose (LNnT) at daily doses up to 20 g to healthy adults modified the gut microbiota with the primary impact being substantial increases in relative abundance of Actinobacteria and *Bifidobacterium* in particular and a reduction in relative abundance of Firmicutes and Proteobacteria. This modulation occurred within 1- 2 weeks (Elison et al. 2016).

In another embodiment, the composition according to the first aspect comprises one or more HMOs selected from 2-fucosyllactose (2'-FL), 3-fucosyllactose (3-FL), difucosyllactose (DFL), lacto-N-tetraose (LNT), lacto-N-neotetraose (LNnT), 3-sialyllactose (3'-SL), 6-sialyllactose (6'-SL), lacto-N-fucopentaose 1 (LNFP-1) or a mixture thereof.

Preferably, the one or more HMOs comprises, consists of or essentially consists of 2'-FL and at least one of LNnT and LNT; at least one of 2'-FL and DFL and at least one of LNnT and LNT (e.g. 2'-FL, DFL and at least one of LNnT and LNT); 2'FL and 6'-SL; DFL and 6'-SL; 2'-FL, DFL and 6'-SL; 2'-FL, 6'-SL and at least one of LNnT and LNT; or 2'-FL, DFL, 6'-SL and at least one of LNnT and LNT.

In some embodiments the composition according to the first aspect is administered to a human subject who is a non-producer as discussed hereinabove. In some embodiments, administration of a composition according to the first aspect of the invention to a human subject of the non-producer group results over time in an alteration of the gut microbiota of said human subject, which is capable of improved catabolization of ellagitannins and/or ellagic acid into one or more of the final urolithins.

Specific embodiments relate to the use of a composition as disclosed herein for enhancing urolithin production by administration to a human subject for a duration of at least 30 days, such as at least 50 days, such as at least 100 days, such as at least 150 days. Preferably, the composition is administered at least until the subject produces one or more final urolithins.

The present composition may be formulated in a formulation, preferably an ingestible formulation, such as a medicament, a dietary supplement, a food additive or a medical food or a food for special medical purpose. The formulation may comprise other ingredients, such as, but not limited to, an excipient, a coating agent, a flavouring agent, one or more herbal extracts or preparations, one or more nutrients, such as one or more vitamins and/or minerals. The composition may be formulated as a tablet, a pill, a capsule, a powder or a granulate. Other formulations suited for the purpose will be obvious to the skilled man and are also envisioned. The composition may be formulated as distinct formulations, wherein the first device comprises the preparation of the fruit of *Punica granatum* and the second device comprises the one or more HMOs. Alternatively, the composition may be formulated as a single formulation.

By analysing the level of urolithins produced by human gut microbiota, the inventors have found a synergistic effect of an HMO blend + a preparation of the fruit of *Punica granatum* compared with administering an HMO blend or a preparation of the fruit of *Punica granatum* individually regarding the final urolithins, urolithin A and iso-urolithin A, and the intermediary urolithin, urolithin C (see Example 5).

In a preferred embodiment, the present invention relates to a method for increasing the production of one or more of urolithin A, urolithin B and isourolithin A in the gut of a human subject, said method comprising administering a composition according to the first aspect as described above to a human subject, in particular a middle-aged or elderly (50+) human subject.

The preparation of the fruit of *Punica granatum* comprises preferably at least 40% polyphenols, at least 30% punicosides and at least 20% punicalagins. In some embodiments, the composition is administered to a human subject at a dosage of at least 75 mg punicalagins/day, such as between 75 mg/day and 1500 mg/day, such as between 100 mg/day and 1000 mg/day, preferably at least 300 mg/day. In some embodiments, the dosage of punicosides is at least 100 mg/day, such as between 100 mg/day and 2000 mg/day, such as between 100 mg/day and 1200 mg/day, preferably at least 400 mg/day. The dosage of polyphenols is at least 125 mg/day, such as between 125 mg/day and 2500 mg/day, such as between 250 mg/day and 1500 mg/day, preferably at least 500 mg/day.

In a preferred embodiment the composition of the present invention may be formulated as a kit comprising ¹⁾ tablets containing pomegranate extract, ²⁾ a powder or granulate combination of the two HMOs 2'-Fucosyllactose (2'-FL) and Lacto-N-neotetraose (LNnT) and ³⁾ instructions for use.

In another embodiment the composition of the present invention may be formulated as tablets comprising a mixture of pomegranate extract, 2'-Fucosyllactose (2'-FL) and Lacto-N-neotetraose (LNnT).

In another embodiment the composition of the present invention may be formulated as a kit comprising ¹⁾ tablets containing pomegranate extract, ²⁾ a powder or granulate of one or more HMOs selected from 2'-FL, 3-FL, DFL, LNT, LNnT, 3'-SL, 6'-SL, LNFP-1 or a mixture thereof, and³⁾ instructions for use.

In another embodiment the composition of the present invention may be formulated as tablets comprising a mixture of pomegranate extract and one or more HMOs selected from 2'-FL, 3-FL, DFL, LNT, LNnT, 3'-SL, 6'-SL, LNFP-1 or a mixture thereof.

In other embodiments of the present invention, the pomegranate extract may be replaced by other natural sources of ellagitannins and/or ellagic acid, such as walnut, raspberry, strawberry and blackberry.

### EXAMPLES

### Example 1

### Formulation of a kit comprising a pomegranate extract and a combination of HMOs

A kit consisting of a pomegranate extract and a combination of the two HMOs 2'-Fucosyllactose (2'-FL) and Lacto-N-neotetraose (LNnT) was developed. The kit consisted of a tablet formulation and a powder formulation.

A dry ethanolic extract of *P. granatum* was used for the tablet formulation. The ethanolic extract was based on pomegranate fruit pomace (Monteloeder S.L., Elche, Alicante, Spain). The content of punicalagins was min. 30%. The daily dose of the pomegranate extract was 1000 mg/4 tablets.

The following tablet excipients were added to the formulation: microcrystalline cellulose, cross-linked sodium carboxymethyl cellulose, sodium carboxymethyl cellulose, silicon dioxide, magnesium salts of vegetable fatty acids, hydroxypropyl methyl cellulose, polyethylene glycol.

As the other active ingredient, a blend of the two HMOs 2'-Fucosyllactose and Lacto-N-neotetraose (Glycom A/S, Hørsholm, Denmark) was used for a powder formulation. The mass ratio between the two HMOs was 2:1 (2'-FL/LNnT). The daily dose of the 2'-FL/LNnT-blend was 5000 mg/stick pack. The following excipient was added to the formulation: magnesium stearate (0.25 mass %).

### Example 2

### Diagnosis of non-producers by urine sample

Non-producers, i.e. individuals who are not capable of producing final urolithins in a total amount higher than 5 µg/mg creatinine in urine, obviously do not benefit from the health benefits related to stimulation and improvement of mitochondrial function conferred by these urolithins. Non-producers are diagnosed by the following method:
A daily dose of pomegranate extract (2 g/8 tablets) containing 30% punicalagins is consumed by the individual for three consecutive days. In the morning of the fourth day, a urine sample is collected and the urolithin content is analysed by LC-MS/MS analysis.

This allows the classification of the individual according to capacity to metabolize ellagitannins into final urolithins. If the total amount of final urolithins is less than 5 µg/mg creatinine in urine, the individual is classified as a non-producer. The exclusive presence of intermediary urolithins does not change the status of non-producer.

### Example 3

### Determination of beneficial HMOs for final urolithin production by a gastrointestinal simulation model

The impact of pomegranate extract combined with one or more HMOs on gut microbiome was investigated in the M-TripleSHIME^{™} *in vitro* gastrointestinal model (Prodigest). The typical reactor setup of the M-TripleSHIME^{™} consists of a succession of four reactors simulating the different parts of the human gastrointestinal tract. The first two reactors are of the fill-and-draw principle to simulate different steps in food uptake and digestion, with peristaltic pumps adding a defined amount of SHIME feed (140 ml 3x/day) and pancreatic and bile liquid (60 ml 3x/day), respectively, to the stomach and small intestine compartment and emptying the respective reactors after specified intervals. The last two compartments are continuously stirred reactors with constant volume and pH control. The retention time and pH of the different vessels are chosen to resemble *in vivo* conditions in the different parts of the colon. The proximal colon is set to pH 5.4-5.6 and retention time= 12 h, and the distal colon is set to pH 6.0-6.5 and retention time= 20 h.

Pomegranate extract with a minimum content of 30% punicalagins and a mixture of 2'-Fucosyllactose and Lacto-N-neotetraose (2'-FL and LNnT) in 2:1 mass ratio is added to the SHIME feed in a concentration that equals 5 grams per day of each ingredient.

Upon inoculation with fecal microbiota from a non-producer individual, these reactors simulate the proximal, transverse and distal colon. After a two-week adaptation of the microbial communities in the different regions of the colon, a representative microbial community is established in the three colon compartments, which differs both in composition and functionality in the different colon regions.

Further, porcine mucin is included in the reactors simulating the colon to take into account the colonisation of the mucous layer. Thus, the M-TripleSHIME^{™} permits culturing both the luminal and mucous-associated microbial community over periods of several weeks.

The M-TripleSHIME^{™} is run in four stages:
1. Stabilization: After inoculation of the reactors with a fresh fecal sample provided by a healthy non-producer, a two-week stabilization period allows the microbial community to differentiate in the different reactors depending on the local environmental conditions.
   During this period, the basic nutritional matrix is provided to support the maximum diversity of the gut microbiota originally present in the fecal inoculum.
2. Control: During this two-week period, a standard nutrient matrix is dosed into the model for a period of 14 days. The baseline microbial community composition and activity in the different reactors is determined by analysis of samples and is used as a reference.
3. Treatment - part 1: The SHIME system is operated under normal conditions for 3 weeks, but with the standard nutrient matrix supplemented with pomegranate extract (min. 30% punicalagins) and the HMOs. The HMOs tested is a 2:1 mixture of 2'-FL and LNnT.
4. Treatment - part 2: The SHIME system is operated under normal conditions for 3 weeks, but with the standard nutrient matrix supplemented with pomegranate extract (min. 30% punicalagins) only.
5. Washout: During this two-week period, the SHIME system is run again with the standard nutrient matrix only.

Sample of the liquids in each reactor are collected regularly and are analysed for the composition of the produced urolithins and the composition of the resident microbial community using 16S rRNA sequencing.

The results from the fermentation system show that final urolithins, especially urolithin A, are produced upon 3 weeks treatment with pomegranate extract and an HMO mixture of 2'-FL and LNnT (2:1). Furthermore, this effect was maintained during the subsequent 3 weeks' treatment with pomegranate extract only, suggesting that a balance had been reached in the gut microbiota towards final urolithin production.

The profiling of the microbial community showed that during the first treatment period the abundance of *Akkermansia* increased in the distal part of the colon regions. Practically no such change occurred in the proximal part. The effect was maintained in the second treatment period.

This *in vitro* study shows that feeding the M-TripleSHIME^{™} with pomegranate extract and the HMO-blend 2'-FL and LNnT (2:1) activated the production of final urolithins in a gut microbiota originating from a non-producer. Furthermore, the effect was maintained during the 3 weeks with pomegranate extract added as the sole supplement to the nutrient matrix. Thus, the gut microbiota of a non-producer may be altered towards the gut microbiota of a urolithin producer by treatment with pomegranate extract and an HMO-blend of 2'-FL and LNnT.

### Example 4

### Treatment of non-producers with a combination of an ellagitannin-rich food source and an HMO blend

The following intervention study was designed to investigate whether a combination of an ellagitannin-rich food source and an HMO blend was able to activate the production of one or more final urolithins by the gut microbiota in non-producers.

This clinical study was a two-part study. In the first part of the study, the urolithin metabotype of a group of potential study participants were determined. Only non-producers were recruited to the second part of the study. In the second part of the study, the effect of 3 weeks' daily consumption of pomegranate extract on urolithin production was determined for each study participant, followed by a comparison of
1) the effect of continued daily consumption of pomegranate extract on urolithin production, with
2) the effect of daily consumption of a combination of pomegranate extract plus an HMO blend on urolithin production.

The dose and composition of the HMO blend was based on a preliminary *in vitro* study.

### Part 1 Metabotype identification

Sixty healthy volunteers (30 males and 30 females) were recruited for this metabotype identification study. Exclusion criteria were: a history of gastrointestinal disease or any chronic disease, following a weight-reducing diet, ongoing treatment with dietary supplements, intake up antibiotics, prebiotics, probiotics or medication up to 8 weeks before the study, pregnancy or lactation. All of the subjects filled out a questionnaire about their dietary habits prior to participation in the study. The intake of ellagitannin-containing sources such as berries (strawberry, raspberry, blackberry etc. and derived foodstuffs such as jam), pomegranates, chocolate, nuts and wine were forbidden for 1 week before the study and during the study (part 1).

A daily dose of pomegranate extract (2 g/8 tablets) containing 40% punicalagins was consumed by the study participant for three consecutive days. In the morning of the fourth day, a urine sample was collected and the urolithin content was analyzed by LC-MS/MS analysis.

The study participants were categorized as UA-producers, mixed producers or non-producers.

Only non-producers were recruited to the second part of the study.

### Part 2 Determination of the effect of pomegranate extract vs. pomegranate extract + HMO blend on urolithin production in non-producers

The non-producers from part 1 were randomized to the following two treatments:
1. Three weeks' treatment with pomegranate extract whereupon urolithin production was tested by a urine test, followed by another three weeks' treatment with pomegranate extract (PE+PE).
2. Three weeks' treatment with pomegranate extract whereupon urolithin production was tested by a urine test, followed by three weeks' treatment with pomegranate extract and an HMO blend of 2'-FL and LNnT (PE+HMO).

A dry ethanolic extract of *P. granatum* was used for a tablet formulation. The ethanolic extract was based on pomegranate fruit pomace (Monteloeder S.L., Elche, Alicante, Spain). The content of punicalagins was 40%. The daily dose of pomegranate extract was 1000 mg/4 tablets.

A blend of the two HMOs 2'-Fucosyllactose and Lacto-N-neotetraose (2'-FL and LNnT) in the mass ratio 2:1 was administered as a powder. The daily dose of the blend was 5000 mg.

After 3 weeks' and 6 weeks' treatment, a daily dose of pomegranate extract (2 g/8 tablets) containing 40% punicalagins was consumed by the study participant for three consecutive days. In the morning of the fourth day, a urine sample was collected and the urolithin content was analyzed by LC-MS/MS analysis.

In week 3 and 6 of the study period (i.e. the week preceding pomegranate extract administration and urine collection), the intake of ellagitannin-containing sources such as berries (strawberry, raspberry, blackberry etc. and derived foodstuffs such as jam), pomegranates, chocolate, nuts and wine were forbidden.

Final urolithins were measured in urine samples for some of the participants after 6 weeks' treatment. Significantly more non-producers had become producers in the PE+HMO group compared with the PE+PE group (*P < 0.05*). Thus, the hypothesis that the combination of pomegranate extract plus HMO activates the production of final urolithins in non-producers, to a higher degree than pomegranate extract alone, was substantiated.

No side effects were reported during the study.

### Example 5

### Stimulation of urolithin production in vitro with a combination of an ellagitannin-rich food source and an HMO blend

The following study was designed to assess the impact of an HMO blend, a pomegranate extract (PG) and the combination of both on the urolithin production by the gut microbiota of nine randomly selected human donors in a short-term *in vitro* simulation. The company ProDigest's (https://www.prodigest.eu/en) short-term colonic simulation model was used to study the interaction between test products and the gut microbiome.

All donors were between 50 and 70 years old, healthy and with no history of antibiotic use 6 months before the experiment. Fecal material was collected, and fecal suspensions prepared and mixed with a cryoprotectant. The obtained suspensions were aliquoted, flash frozen and then preserved at -80°C. Just before the experiment, fecal samples were defrosted and immediately added to the reactors.

The reactors were incubated for 48 hours (37°C, shaking at 90 rpm, anaerobic conditions) with a sugar-depleted nutritional medium containing basal nutrients of the colon. Samples from the reactors were collected at 0h (baseline), after 24 h and after 48h (follow-up). Each fecal sample was used for 4 different treatments:
- Control treatment (blank): only nutritional medium was added to the reactor.
- Human milk oligosaccharide (HMO) treatment: 3 g/l of an HMO blend was added to the reactor at baseline.
- Pomegranate extract (PG) treatment: 1 g/l was added to the reactor at baseline.
- Pomegranate extract + HMO (PG + HMO) treatment: 1g/l of PG and 3 g/l of an HMO blend was added to the reactor at baseline.

Incubations were performed in single repetition, resulting in 36 independent incubations.

The pomegranate extract was an ethanolic extract based on pomegranate fruit pomace (Monteloeder S.L., Elche, Alicante, Spain). The content of punicalagins was approx. 40%.

The HMO blend was a powder consisting of 2'-fucosyllactose (2'FL) and lacto-N-neotetraose (LNnT) in the mass ratio 4:1 (2'FL:LNnT). The HMO blend was provided by Glycom A/S, Hørsholm, Denmark.

The concentration of the final urolithins urolithin A, isourolithin A and urolithin B was measured at 0h, 24h and 48h. Furthermore, the concentration of the intermediary urolithins urolithin C and urolithin D was measured at 0h, 24h and 48h.

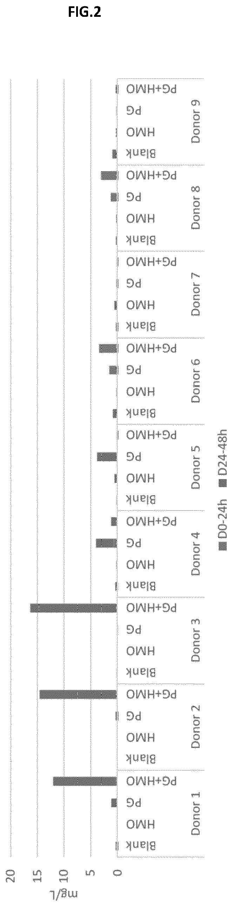

A synergistic effect of HMO+PG treatment compared with HMO treatment or PG treatment, respectively, was seen for urolithin A production in donors 1, 2 and 3 and to a lesser extent in donors 6 and 8 (Figure 2).

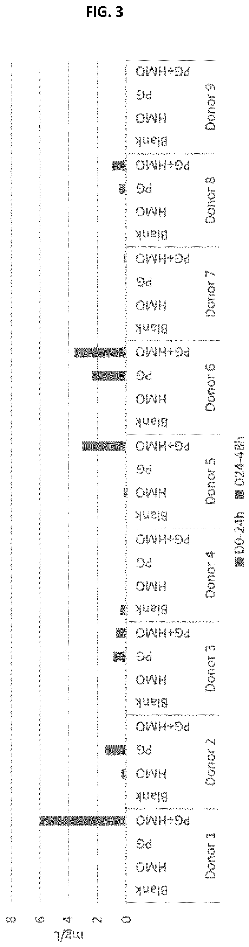

A synergistic effect of HMO+PG treatment compared with HMO treatment or PG treatment, respectively, was seen for isourolithin A production in donors 1 and 5 and to a lesser extent in donors 6 and 8 (Figure 3).

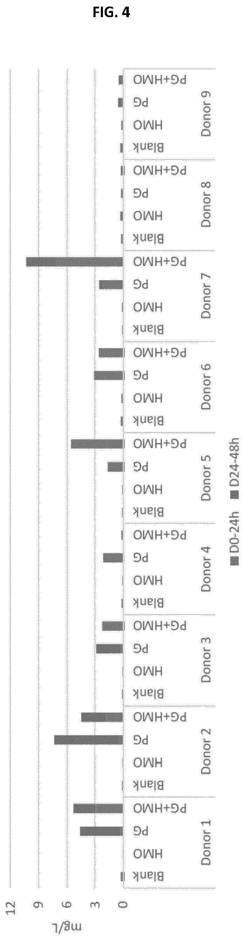

A synergistic effect of HMO+PG treatment compared with HMO treatment or PG treatment, respectively, was seen for urolithin C production in donors 5 and 7 and to a lesser extent in donor 1 (Figure 4).

A synergistic effect of HMO+PG treatment was not observed for urolithin D production.

Urolithin B was not detected in any of the samples. This may be explained by the late synthesis of this compound in the metabolic pathway of final urolithins (Figure 1).

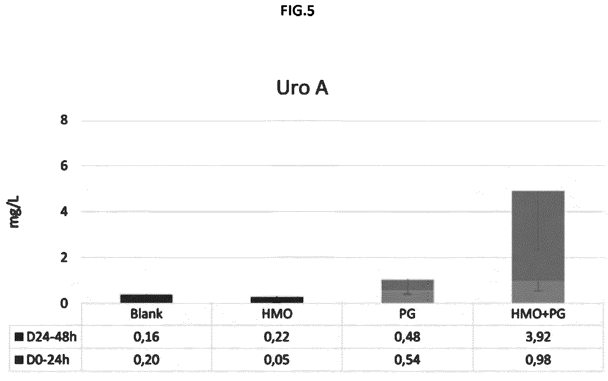

Thus, overall the combination of HMO+PG treatment improved urolithin production in a synergistic manner in fecal sample incubations originating from 7 out of 9 randomly assigned donors (i.e. all donors but donors 4 and 9). The effect was most pronounced for the production of urolithin A. The average data for urolithin A production across the 9 donors are presented in Figure 5.

It is noted that any stimulating effect on non-producers (possibly donors 7 and 9) cannot be expected during a 48h *in vitro* study, since adequate amounts of relevant bacteria for the catabolization of ellagitannins/ellagic acid would probably need weeks to proliferate to a sufficient level.

### References

Andreux PA, Blanco-Bose W, Ryu D, Burdet F, Ibberson M, Aebischer P, Auwerx J, Singh A, Rinsch C (2019) The mitophagy activator urolithin A is safe and induces a molecular signature of improved mitochondrial and cellular health in humans. Nature Metabolism 1 (6):595-603. doi:10.1038/s42255-019-0073-4
Ávila-Gálvez MÁ, Giménez-Bastida JA, Espín JC, González-Sarrías A (2020) Dietary Phenolics against Breast Cancer. A Critical Evidence-Based Review and Future Perspectives. International journal of molecular sciences 21 (16):5718
Cerdá B, Espín JC, Parra S, Martinez P, Tomás-Barberán FA (2004) The potent in vitro antioxidant ellagitannins from pomegranate juice are metabolised into bioavailable but poor antioxidant hydroxy-6H-dibenzopyran-6-one derivatives by the colonic microflora of healthy humans. European journal of nutrition 43 (4):205-220
Cortés-Martín A, Romo-Vaquero M, García-Mantrana I, Rodriguez-Varela A, Collado MC, Espín JC, Selma MV (2019) Urolithin metabotypes can anticipate the different restoration of the gut microbiota and anthropometric profiles during the first year postpartum. Nutrients 11 (9):2079
Djedjibegovic J, Marjanovic A, Panieri E, Saso L (2020) Ellagic Acid-Derived Urolithins as Modulators of Oxidative Stress. Oxidative Medicine and Cellular Longevity 2020
Drummond MJ, Addison O, Brunker L, Hopkins PN, McClain DA, LaStayo PC, Marcus RL (2014) Downregulation of E3 ubiquitin ligases and mitophagy-related genes in skeletal muscle of physically inactive, frail older women: a cross-sectional comparison. Journals of Gerontology Series A: Biomedical Sciences and Medical Sciences 69 (8):1040-1048
Elison E, Vigsnaes LK, Krogsgaard LR, Rasmussen J, Sørensen N, McConnell B, Hennet T, Sommer MO, Bytzer P (2016) Oral supplementation of healthy adults with 2'-O-fucosyllactose and lacto-N-neotetraose is
well tolerated and shifts the intestinal microbiota. British Journal of Nutrition 116 (8):1356-1368 Fang EF, Hou Y, Palikaras K, Adriaanse BA, Kerr JS, Yang B, Lautrup S, Hasan-Olive MM, Caponio D, Dan X (2019) Mitophagy inhibits amyloid-β and tau pathology and reverses cognitive deficits in models of Alzheimer's disease. Nature neuroscience 22 (3):401-412
García-Mantrana I, Calatayud M, Romo-Vaquero M, Espín JC, Selma MV, Collado MC (2019) Urolithin Metabotypes Can Determine the Modulation of Gut Microbiota in Healthy Individuals by Tracking Walnuts Consumption over Three Days. Nutrients 11 (10):2483
Garcia-Villalba R, Vissenaekens H, Pitart J, Romo-Vaquero M, Espín JC, Grootaert C, Selma MV, Raes K, Smagghe G, Possemiers S (2017) Gastrointestinal simulation model TWIN-SHIME shows differences between human urolithin-metabotypes in gut microbiota composition, pomegranate polyphenol metabolism, and transport along the intestinal tract. Journal of agricultural and food chemistry 65 (27):5480-5493
González-Sarrías A, Garcia-Villalba R, Romo-Vaquero M, Alasalvar C, Örem A, Zafrilla P, Tomás-Barberán FA, Selma MV, Espín JC (2017) Clustering according to urolithin metabotype explains the interindividual variability in the improvement of cardiovascular risk biomarkers in overweight-obese individuals consuming pomegranate: A randomized clinical trial. Molecular nutrition & food research 61 (5):1600830
Haas RH (2019) Mitochondrial Dysfunction in Aging and Diseases of Aging. Biology 8 (2). doi:https://doi.org/10.3390/biology8020048
Li Z, Henning SM, Lee R-P, Lu Q-Y, Summanen PH, Thames G, Corbett K, Downes J, Tseng C-H, Finegold SM (2015) Pomegranate extract induces ellagitannin metabolite formation and changes stool microbiota in healthy volunteers. Food & function 6 (8):2487-2495
Nuñez-Sánchez MA, Garcia-Villalba R, Monedero-Saiz T, Garcia-Talavera NV, Gómez-Sánchez MB, Sánchez-Álvarez C, Garcia-Albert AM, Rodriguez-Gil FJ, Ruiz-Marín M, Pastor-Quirante FA (2014) Targeted metabolic profiling of pomegranate polyphenols and urolithins in plasma, urine and colon tissues from colorectal cancer patients. Molecular nutrition & food research 58 (6):1199-1211
Romo-Vaquero M, Garcia-Villalba R, González-Sarrías A, Beltrán D, Tomás-Barberán FA, Espín JC, Selma MV (2015) Interindividual variability in the human metabolism of ellagic acid: Contribution of Gordonibacter to urolithin production. Journal of Functional Foods 17:785-791
Ryu D, Mouchiroud L, Andreux PA, Katsyuba E, Moullan N, Nicolet-dit-Félix AA, Williams EG, Jha P, Sasso GL, Huzard D (2016) Urolithin A induces mitophagy and prolongs lifespan in C. elegans and increases muscle function in rodents. Nature medicine 22 (8):879
Srivastava S (2017) The mitochondrial basis of aging and age-related disorders. Genes 8 (12):398 Tomas-Barberan FA, Garcia-Villalba Ro, Gonzalez-Sarrias A, Selma MV, Espin JC (2014) Ellagic acid metabolism by human gut microbiota: consistent observation of three urolithin phenotypes in intervention trials, independent of food source, age, and health status. Journal of agricultural and food chemistry 62 (28):6535-6538
Truchado P, Larrosa M, García-Conesa MT, Cerdá Ba, Vidal-Guevara ML, Tomás-Barberán FA, Espín JC (2012) Strawberry processing does not affect the production and urinary excretion of urolithins, ellagic acid metabolites, in humans. Journal of Agricultural and Food Chemistry 60 (23):5749-5754

## Claims

1. A composition comprising:
a. a preparation of the fruit of *Punica granatum,* and
b. one or more human milk oligosaccharides (HMOs).

2. A composition according to claim 1 wherein the HMO is selected from neutral HMOs or acidic HMOs.

3. A composition according to any one of claim 1-2 wherein the HMO is one or more fucosylated HMOs or one or more non-fucosylated HMOs, or a mixture of fucosylated and non-fucosylated HMOs.

4. A composition according to any one of claim 1-3 wherein the one or more HMOs is selected from 2'-FL, 3-FL, DFL, LNT, LNnT, 3'-SL, 6'-SL, LNFP-1 or a mixture thereof.

5. A composition according to any one of claim 1-4 wherein the one or more HMOs comprises, consists of or essentially consists of:
a. 2'-FL and at least one of LNnT and LNT;
b. at least one of 2'-FL and DFL and at least one of LNnT and LNT;
c. 2'FL and 6'-SL;
d. DFL and 6'-SL;
e. 2'-FL, DFL and 6'-SL;
f. 2'-FL, 6'-SL and at least one of LNnT and LNT; or
g. 2'-FL, DFL, 6'-SL and at least one of LNnT and LNT.

6. A composition according to any one of claim 1-5 which contains a preparation of the fruit of *Punica granatum* and one or more human milk oligosaccharides (HMOs) in a ratio (w/w) of from 5:1 to 1:10.

7. A composition according to any one of claim 1-6 formulated as a single preparation.

8. A composition according to any one of claim 1-6 formulated as a kit comprising
a. a preparation of the fruit of *Punica granatum,* and
b. one or more human milk oligosaccharides (HMOs),
as separate components, together with instructions for use.

9. A composition according to any one of claim 1-8 for use in the treatment of a human subject identified as being a urolithin non-producer, wherein said use comprises an oral administration of said composition in a daily dose containing between 500 - 10000 mg HMOs/day and 500 - 5000 mg of a preparation of the fruit of *Punica granatum*/day.

10. A composition according to claim 9 for use in the treatment of a human subject identified as being a urolithin non-producer, wherein said daily dose of a preparation of the fruit of *Punica granatum* contains at least 75 mg punicalagins/day, such as between 75 mg/day and 1500 mg/day, such as between 100 mg/day and 1000 mg/day, preferably at least 300 mg/day.

11. A composition according to any one of claim 9 or 10 for use in the treatment of a human subject identified as being a urolithin non-producer, wherein said use comprises an oral administration of said composition, containing between 500 mg and 10000 mg HMOs/day, such as at least 500 mg HMOs/day, such as at least 750 mg HMOs/day, such as at least 1000 mg HMOs/day, such as at least 2000 mg/day such as at least 5000 mg HMOs/day.

## Patentansprüche

1. Zusammensetzung, umfassend:
a. eine Zubereitung der Frucht von Punica granatum und
b. ein oder mehrere menschliche Milcholigosaccharide (HMOs).

2. Zusammensetzung nach Anspruch 1, wobei das HMO aus neutralen HMOs oder sauren HMOs ausgewählt ist.

3. Zusammensetzung nach einem der Ansprüche 1-2, wobei es sich bei dem HMO um ein oder mehrere fucosylierte HMOs oder ein oder mehrere nicht fucosylierte HMOs oder eine Mischung von fucosylierten und nicht fucosylierten HMOs handelt.

4. Zusammensetzung nach einem der Ansprüche 1-3, wobei das eine oder die mehreren HMOs aus 2'-FL, 3-FL, DFL, LNT, LNnT, 3'-SL, 6'-SL, LNFP-1 oder einer Mischung davon ausgewählt sind.

5. Zusammensetzung nach einem der Ansprüche 1-4, wobei das eine oder die mehreren HMOs Folgendes umfassen, daraus bestehen oder im Wesentlichen daraus bestehen:
a. 2'-FL und mindestens eines aus LNnT und LNT;
b. mindestens eines aus 2'-FL und DFL und mindestens eines aus LNnT und LNT;
c. 2'FL und 6'-SL;
d. DFL und 6'-SL;
e. 2'-FL, DFL und 6'-SL;
f. 2'-FL, 6'-SL und mindestens eines aus LNnT und LNT; oder
g. 2'-FL, DFL, 6'-SL und mindestens eines aus LNnT und LNT.

6. Zusammensetzung nach einem der Ansprüche 1-5, die eine Zubereitung der Frucht von Punica granatum und ein oder mehrere menschliche Milcholigosaccharide (HMOs) in einem Verhältnis (Gew./Gew.) von 5:1 bis 1:10 enthält.

7. Zusammensetzung nach einem der Ansprüche 1-6, formuliert als eine einzige Zubereitung.

8. Zusammensetzung nach einem der Ansprüche 1-6, formuliert als Kit, umfassend
a. eine Zubereitung der Frucht von Punica granatum und
b. ein oder mehrere menschliche Milcholigosaccharide (HMOs)
als getrennte Komponenten, zusammen mit Gebrauchsanweisungen.

9. Zusammensetzung nach einem der Ansprüche 1-8 zur Verwendung bei der Behandlung eines als Urolithin-Nichtproduzent identifizierten Menschen, wobei die Verwendung eine orale Verabreichung der Zusammensetzung in einer Tagesdosis umfasst, die zwischen 500-10000 mg HMOs/Tag und 500-5000 mg einer Zubereitung der Frucht von Punica granatum/Tag enthält.

10. Zusammensetzung nach Anspruch 9 zur Verwendung bei der Behandlung eines als Urolithin-Nichtproduzent identifizierten Menschen, wobei die Tagesdosis einer Zubereitung der Frucht von Punica granatum mindestens 75 mg Punicalagine/Tag, wie etwa zwischen 75 mg/Tag und 1500 mg/Tag, wie etwa zwischen 100 mg/Tag und 1000 mg/Tag, vorzugsweise mindestens 300 mg/Tag, enthält.

11. Zusammensetzung nach einem der Ansprüche 9 oder 10 zur Verwendung bei der Behandlung eines als Urolithin-Nichtproduzent identifizierten Menschen, wobei die Verwendung eine orale Verabreichung der Zusammensetzung umfasst, die zwischen 500 mg und 10000 mg HMOs/Tag, wie etwa mindestens 500 mg HMOs/Tag, wie etwa mindestens 750 mg HMOs/Tag, wie etwa mindestens 1000 mg HMOs/Tag, wie etwa mindestens 2000 mg HMOs/Tag, wie etwa mindestens 5000 mg HMOs/Tag enthält.

## Revendications

1. Composition comprenant :
a. une préparation du fruit de Punica granatum, et
b. un ou plusieurs oligosaccharides de lait humain (HMO) .

2. Composition selon la revendication 1, dans laquelle l'HMO est choisi parmi les HMO neutres ou les HMO acides.

3. Composition selon l'une quelconque des revendications 1-2, dans laquelle l'HMO est un ou plusieurs HMO fucosylés ou un ou plusieurs HMO non fucosylés, ou un mélange d'HMO fucosylés et non fucosylés.

4. Composition selon l'une quelconque des revendications 1-3, dans laquelle le ou les HMO sont choisis parmi 2'-FL, 3-FL, DFL, LNT, LNnT, 3'-SL, 6'-SL, LNFP-1 ou un mélange de ceux-ci.

5. Composition selon l'une quelconque des revendications 1-4, dans laquelle le ou les HMO comprennent, sont constitués de ou sont essentiellement constitués de :
a. 2'-FL et au moins l'un parmi LNnT et LNT ;
b. au moins l'un parmi 2'-FL et DFL et au moins l'un parmi LNnT et LNT ;
c. 2'FL et 6'-SL ;
d. DFL et 6'-SL ;
e. 2'-FL, DFL et 6'-SL ;
f. 2'-FL, 6'-SL et au moins l'un parmi LNnT et LNT ; ou
g. 2'-FL, DFL, 6'-SL et au moins l'un parmi LNnT et LNT.

6. Composition selon l'une quelconque des revendications 1-5 qui contient une préparation du fruit de Punica granatum et d'un ou plusieurs oligosaccharides de lait humain (HMO) dans un rapport (p/p) allant de 5:1 à 1:10.

7. Composition selon l'une quelconque des revendications 1-6 formulée sous la forme d'une préparation unique.

8. Composition selon l'une quelconque des revendications 1-6 formulée sous la forme d'un kit comprenant
a. une préparation du fruit de Punica granatum, et
b. un ou plusieurs oligosaccharides de lait humain (HMO),
en tant que composants distincts, accompagnés d'instructions d'utilisation.

9. Composition selon l'une quelconque des revendications 1-8 destinée à être utilisée dans le traitement d'un sujet humain identifié comme étant un non-producteur d'urolithine, dans laquelle ladite utilisation comprend une administration orale de ladite composition en une dose quotidienne contenant entre 500 et 10 000 mg d'HMO/jour et 500 à 5 000 mg d'une préparation du fruit de Punica granatum/jour.

10. Composition selon la revendication 9, destinée à être utilisée dans le traitement d'un sujet humain identifié comme étant un non-producteur d'urolithine, dans laquelle ladite dose quotidienne d'une préparation du fruit de Punica granatum contient au moins 75 mg de punicalagines/jour, par exemple entre 75 mg/jour et 1 500 mg/jour, par exemple entre 100 mg/jour et 1 000 mg/jour, de préférence au moins 300 mg/jour.

11. Composition selon l'une quelconque des revendications 9 ou 10, destinée à être utilisée dans le traitement d'un sujet humain identifié comme étant un non-producteur d'urolithine, dans laquelle ladite utilisation comprend une administration orale de ladite composition, contenant entre 500 mg et 10 000 mg d'HMO/jour, par exemple au moins 500 mg d'HMO/jour, par exemple au moins 750 mg d'HMO/jour, par exemple au moins 1 000 mg d'HMO/jour, par exemple au moins 2 000 mg d'HMO/jour, par exemple au moins 5 000 mg d'HMO/jour.
